# EUROPÄISCHE PATENTSCHRIFT

(11) **EP 0 968 172 B1**
(45) Veröffentlichungstag und Bekanntmachung des Hinweises auf die Patenterteilung: **12.06.2002**
(21) Anmeldenummer: 98909472.7
(22) Anmeldetag: 21.02.1998
(51) Int. Cl.: C07C 213/00, C07C 215/14

(54) **VERFAHREN ZUR HERSTELLUNG VON N,N,N',N'-TETRA-(2-HYDROXYETHYL)ETHYLENDIAMIN**
PROCESS FOR PREPARING N,N,N',N'-TETRA-(2-HYDROXYETHYL)ETHYLENEDIAMINE
PROCEDE DE PREPARATION DE N,N,N',N'-TETRA-(2-HYDROXYETHYL)ETHYLENEDIAMINE

(30) Priorität: 27.02.1997 DE 19707872
(43) Veröffentlichungstag der Anmeldung: 05.01.2000
(73) Patentinhaber: Akzo Nobel N.V., 6824 BM Arnhem (NL)
(72) Erfinder: IHRIG, Klaus, D-69429 Waldbrunn (DE); BERGFELD, Manfred, D-63906 Erlenbach (DE); BLAUFELDER, Christian, D-63785 Obernburg (DE)
(74) Vertreter: Fett, Günter
(86) Internationale Anmeldenummer: EP9801008
(87) Internationale Veröffentlichungsnummer: WO9838153

(56) Entgegenhaltungen:
- DE-B- 1 020 347
- CHEMICAL ABSTRACTS, vol. 35, no. 1, 1941 Columbus, Ohio, US; abstract no. 5858, XP002064711 in der Anmeldung erwähnt & SHERLIN ET AL.: "The spontaneous condensation of tetra(2-chloroethyl)-ethylenediamnie" J. GEN. CHEM. (USSR), Bd. 11, 1941, Seiten 305-308,

## Beschreibung

Die Erfindung betrifft ein Verfahren zur Herstellung von N,N,N',N'-Tetra-(2-hydroxyethyl)-ethylendiamin (= THEEDA) durch Umsetzung von Ethylendiamin mit Ethylenoxid in einem Lösungsmittel.

Es ist nach der DE-AS 1 020 347 bekannt, Alkylendiamine mit 2 bis 6 Kohlenstoffatomen, z.B. Ethylendiamin, mit Propylenoxid in Anwesenheit von Wasser oder einem Alkohol als Umsetzungskatalysator bei einer Temperatur von 40 bis 200 °C, bevorzugt 40 bis 120 °C, in einem Molverhältnis von 1:4 umzusetzen. Die Reaktion liefert nahezu quantitative Ausbeuten, und diese sind noch nicht einmal davon abhängig, ob mit einem Überschuß an Propylenoxid gearbeitet wird oder nicht, da sich die tetraoxypropylierten Alkylendiamine mit überschüssigem Propylenoxid unter den genannten Reaktionsbedingungen nicht mehr weiter umsetzen.

In der DE-AS 1 020 347 wird hervorgehoben, daß im Gegensatz zum tetraoxypropylierten Ethylendiamin das von Sherlin und anderen nach Chemical Abstracts 35, 5858 (1941) hergestellte N,N,N',N'-Tetra-(2-hydroxyethyl)-ethylendiamin sich beim Erhitzen zersetzt und nicht destilliert werden kann. Die Herstellung des Produktes, das jedoch mit mannigfaltigen Nebenprodukten behaftet ist, erfolgt ebenda durch vierstündiges Stehen von Ethylendiamin und Ethylenoxid in wäßriger Lösung und anschließende Beseitigung des Wassers mittels Vakuum. Die Reaktionsprodukte liegen in Form eines dicken Öls vor.

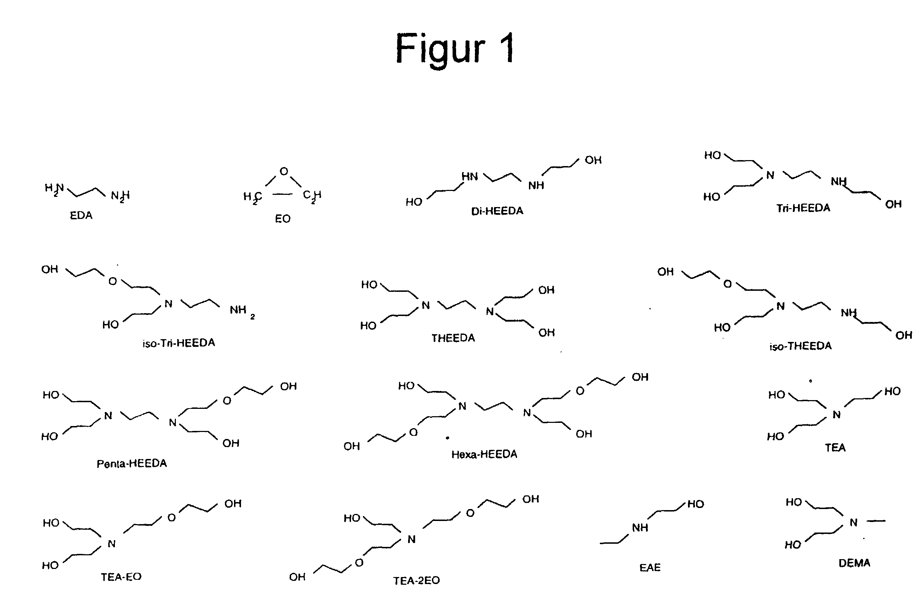

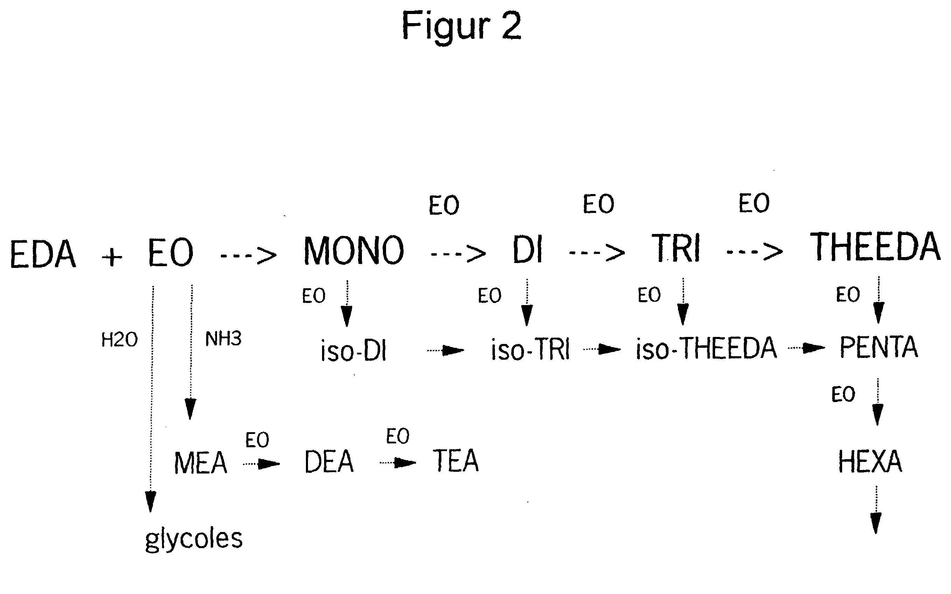

Es stellt eine Tatsache dar, daß das Ethylenoxid gegenüber Ethylendiamin bezüglich der Bildung des tetraoxyethylierten Ethylendiamins unter weiten Reaktionsbedingungen eine gute Selektivität vermissen läßt, wie sie umgekehrt das Propylenoxid bei der Reaktion zum tetraoxypropylierten Ethylendiamin aufweist. Figur 1 zeigt aus einer umfangreichen Forschungsarbeit, die zur vorliegenden Erfindung geführt hat, daß z.B. unter den Bedingungen der DE-AS 1 020 347 neben THEEDA eine Anzahl von möglichen Nebenprodukten gebildet wird, die sich vom di-oxyethylierten Ethylendiamin (= Di-HEEDA) bis zum Ethylaminoethanol (= EAE) und Methyldiethanolamin (= MDEA) erstrecken. Figur 2 faßt in Form eines Diagramms die Reaktionswege zu den entsprechenden Nebenprodukten und dem gewünschten Produkt THEEDA zusammen.

Überraschenderweise kommt es nämlich bei der Einwirkung von Ethylenoxid (= EO) auf Ethylendiamin (= EDA) zu einer Ammoniakabspaltung, die durch Reaktion mit EO die Bildung von Monoethanolamin (= MEA), Diethanolamin (= DEA) und Triethanolamin (= TEA) zur Folge hat. In Anwesenheit von Wasser fallen auch die Glykole Ethylenglykol, Diethylenglykol und Triethylenglykol als Nebenprodukte der EO-Umsetzung an.

Die Möglichkeit eines ökonomischen großtechnischen Verfahrens zur Herstellung von N,N,N',N'-Tetra-(2-hydroxyethyl)-ethylendiamin hängt nach Figur 2 davon ab, ob Reaktionsbedingungen existieren, die die Hauptreaktion von Ethylendiamin mit Ethylenoxid über Mono-HEEDA (= N-Mono-(2-hydroxyethyl)-ethylendiamin), Di-HEEDA (= N,N -Di-(2-hydroxyethyl)-ethylendiamin) oder N,N'-Di(2-hydroxyethyl)-ethylendiamin und Tri-HEEDA (= N,N,N'-Tri-(2-hydroxyethyl)-ethylendiamin) zu THEEDA enorm begünstigen (man beachte, daß in Figur 2 in Zeile 1 die ersten drei genannten Verbindungen mit MONO, DI und TRI abgekürzt worden sind). Dies bedeutet gleichzeitig, daß die in Figur 2 aufgeführten möglichen Nebenreaktionen, die entweder über THEEDA oder auch über die Iso-Verbindungen iso-Di-HEEDA, iso-Tri-HEEDA (in Figur 2 in Zeile 2 mit iso-Di und iso-TRI abgekürzt) und iso-THEEDA zu Penta-HEEDA und Hexa-HEEDA (in Figur 2 rechts mit PENTA und HEXA abgekürzt) führen, nahezu vermieden werden müssen.

Die vorliegende Erfindung stellt sich somit die Aufgabe, ein ökonomisches technisches Verfahren zur Herstellung von N,N,N',N'-Tetra-(2-hydroxyethyl)-ethylendiamin (= THEEDA) in einem Lösungsmittel bereitzustellen, in dem die Ausgangskomponenten Ethylenoxid und Ethylendiamin eine hohe Selektivität zur Bildung von THEEDA aufweisen und demzufolge die Anwesenheit von unterethoxylierten und überethoxylierten Tetra-HEEDA-Derivaten, wie Tri-HEEDA, Penta-HEEDA und Hexa-HEEDA, sowie die Anwesenheit von Mono-, Di- und Triethanolamin, praktisch vermieden wird. Die geforderte hohe Selektivität ist deshalb von entscheidender Bedeutung, weil die genannten Nebenprodukte nicht oder nicht wirtschaftlich vom gewünschten Endprodukt abgetrennt werden können und somit die Produktqualität verschlechtern.

Es wurde nun gefunden, daß man THEEDA in hoher Ausbeute und Selektivität dadurch erhalten kann, daß man Ethylendiamin mit Ethylenoxid in Abwesenheit von Wasser in einem oder mehreren gesättigten C₃-C₉- Alkohol(en) bei einer Temperatur von 120 bis 220°C und einem Druck von 2 bis 60 bar in einem Molverhältnis von 1:4 umsetzt.

Im Gegensatz zu der oben besprochenen Oxypropylierung von Ethylendiamin, wo die Anwesenheit von Wasser sogar erwünscht ist, wäre bei der entsprechenden Oxyethylierung die Verwendung von Wasser als Lösungsmittel mit nicht hinzunehmenden Ausbeuteverlusten verbunden. Wasser führt zur Hydrolye des Endproduktes THEEDA, und es entsteht insbesondere als Nebenprodukt Triethanolamin in unerwünschter Menge. Wie zudem das Vergleichsbeispiel 3 zeigt, wird mit Wasser als Lösungsmittel bei einer Reaktionstemperatzur von 140 °C nur eine THEEDA-Ausbeute von 63% erhalten. Auch die Verwendung von niederen Alkoholen als organische Lösungsmittel, wie Methanol und Ethanol, verhindert die erforderliche hohe Selektivität, wie die Vergleichsbeispiele 4 und 5 zeigen. Der gleiche Sachverhalt ist bei der Verwendung von höheren Alkoholen ab C₁₀ festzustellen (vgl. Vergleichsbeispiel 6).

Die erfindungsgemäß verwendeten organischen Lösungsmittel stellen gesättigte C₃ bis C₉-Alkohole dar. Beispielhaft seien dafür genannt: n-Propanol, Isopropylalkohol, n-Butanol, Isobutanol, tert.-Butylalkohol, 1-Pentanol, 2-Methyl-1-butanol, 3-Methyl-1-butanol, 2,2-Dimethyl-1-propanol, 1-Hexanol, 2-Methyl-1-pentanol, 2-Ethyl-1-butanol, 4-Methyl-2-pentanol, 1-Heptanol, 3-Methyl-1-hexanol, 2-Methyl-2-hexanol, 1-Octanol, 2-Octanol, 2-Ethyl-1-hexanol, 3,3-, 3,5- und 4,5-Dimethyl-1-hexanol, 3-und 5-Methyl-1-heptanol, 1-Nonanol, 2,6-Dimethyl-4-heptanol, 3,5,5-Trimethyl-1-hexanol und Cyclohexanol. Als niederer isomerfreier Alkohol wird der Einsatz von Isopropylalkohol besonders bevorzugt.

Höhere Alkohole, und zwar ab den C₁₀-Alkoholen, verhindern ebenso eine hohe Selektivität, wie die oben schon erwähnten polareren niederen Alkohole oder sehr polare oder unpolare Lösungsmittel schlechthin, beispielsweise Cyclohexan und Diethylether. Grundsätzlich ist es möglich, zur Herstellung von THEEDA auch Gemische der gesättigten C₃ bis C₉-Alkohole einzusetzen. Diese Gemische können im Prinzip beliebige Stoffe und prozentuale Zusammensetzungen aus der Gesamtmenge der gesättigten C₃-C₉-Alkohole enthalten. Im Sinne der Erfindung gelten daher die entsprechenden Alkoholgemische als ein Lösungsmittel.

Die Verwendung von Alkoholgemischen ist aufgrund der bestehenden Isomerieverhältnisse besonders vorteilhaft bei einem Einsatz von höheren Alkoholen. Schon ab den C₅-Alkoholen wird es auch aus ökonomischen Gründen bevorzugt, anstelle der isomerenfreien C₅-Einzelalkohole Isomerengemische der Amylalkohole einzusetzen, wie sie beispielsweise bei der Oxosynthese anfallen. Die Oxo-Alkohole bestehen ausschließlich aus primären Alkoholen. Das nach der Hydroformylierung und Hydrierung von n-Buten erhaltene Alkoholgemisch enthält etwa zwei Drittel 1-Pentanol und ein Drittel 2-Methyl-1-butanol neben geringen Mengen 3-Methyl-1-butanol.Es ist unter der Bezeichnung "Amylalkohol" oder auch "primärer Amylalkohol" käuflich.

Ebenso wird es bevorzugt, C₇-C₉-Alkoholgemische als Lösungsmittel einzusetzen. Diese sind Gemische der C-Zahlen 7 und 8 oder 7 bis 9, die aus geradkettigen Olefinschnitten über die Oxosynthese hergestellt werden. Sie sind unter den Namen Oxanol 78 (Ruhrchemie) und Alphanol 79 (ICI, Shell) erhältlich. Oxanol 78 besteht etwa zu 70-75% aus C₇-Alkoholen und zu 30-25% aus C₈-Alkoholen. Der Anteil an geradkettigen Alkoholen beträgt etwa 60-65%, während die restlichen 35-40% aus vorwiegend methylverzweigten Alkoholen bestehen. Alphanol 79 stellt ein Alkoholgemisch mit etwa 45% C₇-, 43% C₈- und 12% C₉-Alkoholen dar.

Isomere C₈-Alkoholgemische, die bei der Hydroformylierung reiner Heptene oder geeigneter Heptenfraktionen, welche man meistens durch Mischdimerisation von Buten und Propen gewinnt, erhalten werden, und isomere C₉-Alkoholgemische, die man durch Dimerisierung oder Mischdimerisierung von Buten und Isobuten und anschließende Hydroformylierung der C₈-Olefine herstellt, stellen im Rahmen der vorliegenden Erfindung ebenfalls bevorzugte Lösungsmittelgemische dar. Die Reinheit dieser Alkoholgemische sollte stets > 99% betragen.

Ein weiteres wesentliches Merkmal der vorliegenden Erfindung stellt der für eine hohe Selektivität erforderliche Temperatur- und Druckbereich von 120 bis 220 C und 2 bis 60 bar dar. Es hat sich gezeigt, daß die bei der Oxypropylierung von Ethylendiamin bevorzugten niederen Temperaturen von z.B. 70 bis 100 °C (vgl. Beispiele der DE-AS 1 020 347) bei der Oxyethylierung von Ethylendiamin zu niedrigen Ausbeuten und hohen Mengen an Tri-, Penta- und Hexa-HEEDA führen. Dies zeigen die Vergleichsbeispiele 1 und 2. Im Rahmen der Erfindung wird ein Temperaturbereich von 140 bis 180°C bevorzugt.

Das Molverhältnis von Ethylendiamin zu Ethylenoxid von 1:4 muß im Gegensatz zur Oxypropylierung von Ethylendiamin bei der Oxyethylierung von Ethylendiamin sehr genau eingehalten werden. Während bei der Oxypropylierung von Ethylendiamin ein Überschuß an Propylenoxid sogar unschädlich ist und nicht zur Bildung von unerwünschten höheren Oligomeren führt, zeigen die Vergleichsbeispiele 7 und 8 bei der Oxyethylierung von Ethylendiamin genau das gegenteilige Bild. Schon ein 5%iger Ethylenoxid-Überschuß läßt die Ausbeute an THEEDA durch Bildung entsprechender Mengen an Penta-HEEDA und TEA auf 92,2% sinken. Bei einem 5%igen Ethylenoxid-Unterschuß sinkt die Ausbeute an THEEDA durch Bildung vermehrter Mengen an Tri-HEEDA auf 90,5%.

Im allgemeinen werden der oder die C₃-C₉-Alkohol(e) und das Ethylendiamin in dem Reaktionsbehälter vorgelegt, der Reaktionsraum mit Stickstoff gespült und auf die Reaktionstemperatur aufgeheizt, woraufhin dann das Ethylenoxid zudosiert wird. Die Dosierzeit des Ethylenoxids kann in einem weiten Rahmen variiert werden. Im allgemeinen beträgt diese 5 bis 60 Minuten, wobei eine Ethylenoxid-Dosierzeit von 15 bis 25 Minuten in der Regel bevorzugt wird.

Auch die Menge an C₃-C₉-Alkohol(en) kann in weiten Grenzen variiert werden. Sehr hohe Mengen an C₃-C₉-Alkohol(en) führen zwar zu noch höheren Selektivitäten, aber auch zu großen Apparatevolumina, was den Prozeß verteuert. Sehr niedrige Mengen an C₃-C₉-Alkohol(en) lassen die Selektivität wieder etwas absinken und können Probleme bei der Wärmeabfuhr ergeben, so daß gegebenenfalls teure Kühlvorrichtungen bzw. Apparatekonstruktionen erforderlich werden. Es hat sich als vorteilhaft erwiesen, die Menge an C₃-C₉-Alkohol(en) so zu wählen, daß die THEEDA-Konzentration nach der Umsetzung 5 bis 60%, bevorzugt 20 bis 40 % beträgt.

Aufgrund der relativ hohen Reaktionszeiten, wie sie für Ethoxylierungsreaktionen typisch sind, eignet sich das erfindungsgemäße Verfahren auch hervorragend zur kontinuierlichen Prozeßführung in einem Strömungsrohrreaktor oder in einer Kaskade von Rührkesselreaktoren. Das effiziente Reaktionssystem macht es möglich, daß hierbei nur ein geringes Reaktorvolumen benötigt wird, um den vollständigen Umsatz des Ethylendiamins zu Tetra-HEEDA herbeizuführen. Vor allem bei hohen Produktkonzentrationen eignet sich eine Kaskade von Rührkesselreaktoren besonders gut, um einerseits effektiv die entstehende Reaktionswärme abzuführen und um andererseits durch die intensive Vormischung die lokalen Konzentrationen an Ethylenoxid zu minimieren.

Nach der Abtrennung der jeweils angewandten gesättigten C₃-C₉-Alkohole, beispielsweise durch Vakuumdestillation, fällt das nach dem erfindungsgemäßen Verfahren hergestellte THEEDA in einer solchen Qualität an, daß es im Unterschied zum Stand der Technik ohne Zersetzung destillativ gereinigt werden kann (Beispiel 18). Das erfindungsgemäße Verfahren ermöglicht es, großtechnisch ein interessantes Zwischenprodukt bereitzustellen, das aufgrund seiner symmetrischen Struktur und seiner reaktionsfähigen primären Hydroxylgruppen insbesondere zur Herstellung von Hydroxyethylesterquats (Weichmacher), zur Herstellung von crosslinking agents und als Modifizierungskomponente bei der Herstellung von Polymeren, wie Polyestern und Polyurethanen, eingesetzt werden kann.

Die Erfindung wird anhand der nachfolgenden Beispiele näher erläutert.

### Beispiel 1

In einen 35-Liter Edelstahlreaktor mit Rührer und Doppelmantel (Thermostatkühlung/-beheizung) werden 0,326 kg Ethylendiamin (5,42 Mol) und 13,55 Liter Isopropanol gegeben. Der Reaktionsraum wird mit Stickstoff gespült und anschließend auf 140 °C aufgeheizt. Beim Erreichen der Reaktionstemperatur werden innerhalb von 47 Minuten 0,956 kg Ethylenoxid (21,68 Mol) zudosiert. Nach Beendigung der Ethylenoxid-Dosierung wird die Temperatur noch 120 Minuten beibehalten und anschließend der Versuch beendet. Nach dem Abkühlen wird das Lösungsmittel durch Verdampfung im Vakuum vom Reaktionsprodukt abgetrennt. Bei vollständigem Umsatz betrug die Ausbeute an THEEDA 96,3%.

### Beispiel 2

In einen 35-Liter Edelstahlreaktor mit Rührer und Doppelmantel (Thermostatkühlung/-beheizung) werden 13,55 Liter Isopropanol gegeben. Der Reaktionsraum wird mit Stickstoff gespült und anschließend auf 140°C aufgeheizt. Beim Erreichen der Reaktionstemperatur werden innerhalb von 1 Minute 0,326 kg Ethylendiamin (5,42 Mol) und anschließend innerhalb von 57 Minuten 0,956 kg Ethylenoxid (21,68 Mol) zudosiert. Nach Beendigung der Ethylenoxid-Dosierung wird die Temperatur noch 120 Minuten beibehalten und anschließend der Versuch beendet. Nach dem Abkühlen wird das Lösungsmittel durch Verdampfung im Vakuum vom Reaktionsprodukt abgetrennt. Bei vollständigem Umsatz betrug die Ausbeute 96,2%.

### Beispiel 3

In einen 1-Liter Büchi-Edelstahlreaktor mit Rührer und Doppelmantel (Thermostatkühlung/-beheizung) werden 12,02 g Ethylendiamin (0,2 Mol) und 700 ml Isopropanol gegeben. Der Reaktionsraum wird mit Stickstoff gespült und anschließend auf 140 °C aufgeheizt. Beim Erreichen der Reaktionstemperatur werden innerhalb von 25 Minuten 35,24 g Ethylenoxid (0,8 Mol) zudosiert. Nach Beendigung der Ethylenoxid-Dosierung wird die Temperatur noch 120 Minuten beibehalten und anschließend der Versuch beendet. Nach dem Abkühlen wird das Lösungsmittel durch Verdampfung im Vakuum vom Reaktionsprodukt abgetrennt. Bei vollständigem Umsatz betrug die Ausbeute an THEEDA 97,0%.

### Beispiel 4

Beispiel 3 wurde mit zwei Ausnahmen wiederholt. Die Ethylenoxid-Dosierzeit betrug nunmehr 20 Minuten, während die Reaktionszeit nach der Ethylenoxid-Dosierung auf 240 Minuten verlängert wurde. Es wurde hierbei eine THEEDA-Ausbeute von 97,6% erhalten.

### Beispiel 5

In einen 1-Liter Büchi-Edelstahlreaktor, ausgerüstet mit Rührer und Doppelmantel (Thermostatkühlung/-beheizung) werden 12,02 g Ethylendiamin (0,2 Mol) und 500 ml Isopropanol gegeben. Der Reaktionsraum wird wiederum mit Stickstoff gespült und anschliessend auf 160°C aufgeheizt. Beim Erreichen der Reaktionstemperatur werden innerhalb von 25 Minuten 35,24 g Ethylenoxid (0,8 Mol) zudosiert. Danach wird die Temperatur noch 120 Minuten beinehalten und anschließend der Versuch beendet. Nach dem Abkühlen wird das Lösungsmittel wiederum durch Verdampfung im Vakuum vom Reaktionsprodukt abgetrennt. Bei vollständigem Umsatz betrug die Ausbeute an THEEDA 97,9%.

### Beispiel 6

Beispiel 5 wurde mit der einzigen Abänderung wiederholt, daß an Stelle von Isopropanol diesmal n-Propanol als Lösungsmittel eingesetzt wurde. Die Ausbeute an THEEDA betrug 95,9%.

### Beispiel 7

Beispiel 5 wurde mit der einzigen Abänderung wiederholt, daß an Stelle von Isopropanol Cyclohexanol eingesetzt wurde. Die Ausbeute an THEEDA betrug 96,3%.

### Beispiel 8

Beispiel 5 wurde mit der einzigen Abänderung wiederholt, daß an Stelle von Isopropanol tert.-Butanol als Lösungsmittel eingesetzt wurde. Die Ausbeute an THEEDA betrug 96,9%.

### Beispiel 9

Beispiel 5 wurde mit der einzigen Abänderung wiederholt, daß an Stelle von Isopropanol n-Butanol als Lösungsmittel eingesetzt wurde. Die Ausbeute an THEEDA betrug 96,4%.

### Beispiel 10

Beispiel 5 wurde mit der einzigen Abänderung wiederholt, daß an Stelle von Isopropanol Isobutanol als Lösungsmittel verwendet wurde. Die Ausbeute an THEEDA betrug 95,6%.

### Beispiel 11

Beispiel 5 wurde mit der einzigen Abänderung wiederholt, daß an Stelle von 500 ml Isopropanol nunmehr nur 200 ml Isopropanol verwendet wurden. Die Ausbeute an THEEDA betrug 95,9%.

### Beispiel 12

Beispiel 5 wurde mit zwei Abänderungen wiederholt. Die Ethylenoxid-Dosierzeit wurde von 25 Minuten auf 5 Minuten erheblich gekürzt und die Reaktionszeit nach der Zudosierung des Ethylenoxids wurde ebenfalls von 120 Minuten auf 60 Minuten beträchtlich reduziert. Die Ausbeute an THEEDA betrug 97,0%.

### Beispiel 13

Beispiel 5 wurde mit der einzigen Abänderung wiederholt, daß an Stelle einer Ethylenoxid-Dosierzeit von 25 Minuten dieselbige nunmehr auf 63 Minuten verlängert wurde. Die THEEDA-Ausbeute betrug 96,8%.

### Beispiel 14

Beispiel 5 wurde mit der einzigen Abänderung wiederholt, daß als Reaktionstemperatur an Stelle von 160°C nunmehr die von 180 °C gewählt wurde.Die Ausbeute an THEEDA betrug 96,7%.

### Beispiel 15

Beispiel 5 wurde mit der einzigen Abänderung wiederholt, daß als Reaktionstemperatur an Stelle von 160 °C nunmehr die von 120 °C gewählt wurde. Die Ausbeute an THEEDA betrug 94,8%.

### Beispiel 16

Beispiel 5 wurde mit 2 Abänderungen wiederholt. An Stelle von Isopropanol wurde die gleiche g-Menge 2-Ethyl-1-hexanol als organisches Lösungsmittel verwendet, und die Ethylenoxid-Dosierzeit betrug 20 Minuten. Es wurde eine Ausbeute an THEEDA von 97% erhalten.

### Beispiel 17

Beispiel 5 wurde mit drei Abänderungen wiederholt. An Stelle von Isopropanol wurde die gleiche g-Menge 1-Nonanol verwendet. Die Ethylenoxid-Dosierzeit betrug 15 Minuten und die Reaktionszeit nach der Zudosierung des Ethylenoxids 60 Minuten. Es wurde eine Ausbeute an THEEDA von 96% erhalten.

### Beispiel 18

15 g des Reaktionsproduktes aus Beispiel 5, das als Nebenprodukt u.a. 0,5 Gew.% Triethanolamin enthielt, wurde innerhalb von 150 Minuten einer fraktionierten Destillation in einem Kugelrohr-Destillationsapparat unterzogen. Die Temperaturen wurden von 215 °C bis auf 250 °C erhöht bei einem konstanten Druck von 0,01 torr. Nach Beendigung der Destillation konnte das Triethanolamin nahezu vollständig vom höhersiedenden THEEDA abgetrennt werden. Eine Zersetzung des THEEDA fand dabei nicht statt.

### Vergleichsbeispiel 1

Beispiel 5 wurde mit einer Abänderung wiederholt. An Stelle von 160 °C wurde als Reaktionstemperatur 80 °C gewählt. Die Ausbeute an THEEDA betrug 70,6%, und es wurden als Nebenprodukte insbesondere relativ hohe Mengen an Tri-, Penta- und Hexa-HEEDA erhalten.

### Vergleichsbeispiel 2

Beispiel 5 wurde mit einer Abänderung wiederholt. An Stelle von 160°C wurde als Reaktionstemperatur 100°C gewählt. Die Ausbeute an THEEDA betrug 87,8%, und es wurden als Nebenprodukte insbesondere relativ hohe Mengen an Tri-, Penta- und Hexa-HEEDA erhalten.

### Vergleichsbeispiel 3

Beispiel 5 wurde mit zwei Abänderungen wiederholt. An Stelle von 500 ml Isopropanol wurden 500 ml Wasser als Lösungsmittel verwendet und als Reaktionstemperatur wurde die von Beispiel 1, nämlich 140 °C gewählt. Die THEEDA-Ausbeute betrug 63 %.

### Vergleichsbeispiel 4

Beispiel 5 wurde mit zwei Abänderungen wiederholt. An Stelle von 500 ml Isopropanol wurden 500 ml Methanol als Lösungsmittel verwendet und als Reaktionstemperatur wurde die von Beispiel 1, nämlich 140 °C gewählt. Die Ausbeute an THEEDA betrug 79,2 %.

### Vergleichsbeispiel 5

Beispiel 5 wurde mit zwei Abänderungen wiederholt. An Stelle von 500 ml Isopropanol wurden 500 ml Ethanol als Lösungsmittel verwendet und als Reaktionstemperatur wurde die von Beispiel 1, nämlich 140 °C gewählt. Die THEEDA-Ausbeute betrug 79,2 %.

### Vergleichsbeispiel 6

Beispiel 5 wurde mit drei Abänderungen wiederholt. An Stelle von Isopropanol wurde die gleiche g-Menge 1-Decanol verwendet.

Die Ethylenoxid-Dosierzeit betrug 15 Minuten und die Reaktionszeit nach der Zudosierung des Ethylenoxids 60 Minuten. Es wurde eine Ausbeute an THEEDA von nur 90% erhalten, und es ließen sich im Produkt deutlich erhöhte Mengen an TEA und höheren Oligomeren, wie Hexa-HEEDA, nachweisen.

### Vergleichsbeispiel 7

Beispiel 5 wurde mit einer Abänderung wiederholt. An Stelle von 35,24 g bzw. 0,8 Molen Ethylenoxid wurden nunmehr 37,0 g bzw. 0,84 Mole Ethylenoxid, d.h. ein 5%iger Ethylenoxid-Überschuß eingesetzt.Die Ausbeute an THEEDA betrug 92,2%, und es wurden als Nebenprodukte insbesondere relativ hohe Mengen an Penta-HEEDA und TEA (Triethanolamin) erhalten.

### Vergleichsbeispiel 8

Beispiel 5 wurde mit einer Abänderung wiederholt. An Stelle von 35,24 g bzw. 0,8 Molen Ethylenoxid wurden nunmehr 33,56 g bzw. 0,76 Mole Ethylenoxid, d.h. ein 5%iger Ethylenoxid-Unterschuß eingesetzt. Die Ausbeute an THEEDA betrug 90,5 %, und es wurde als Nebenprodukt insbesondere eine relativ hohe Menge an Tri-HEEDA erhalten.

### Vergleichsbeispiel 9

Beispiel 5 wurde mit einer Abänderung wiederholt. Die Abänderung bestand darin, daß überhaupt kein Lösungsmittel verwendet wurde.Die Ausbeute an THEEDA betrug 89,4 %, und es wurden als Nebenprodukte insbesondere relativ hohe Mengen an Penta-HEEDA und Hexa-HEEDA erhalten.

## Patentansprüche

1. Verfahren zur Herstellung von N,N,N',N'-Tetra-(2-hydroxyethyl)-ethylendiamin durch Umsetzung von Ethylendiamin mit Ethylenoxid in einem Lösungsmittel, **dadurch gekennzeichnet, daß** man Ethylendiamin mit Ethylenoxid in Abwesenheit von Wasser in einem oder mehreren gesättigten C₃ - C₉-Alkohol(en) bei einer Temperatur von 120 bis 220°C und einem Druck von 2 bis 60 bar in einem Molverhältnis von 1:4 umsetzt.

2. Verfahren nach Anspruch 1, **dadurch gekennzeichnet, daß** die Umsetzung in Isopropanol erfolgt.

3. Verfahren nach Anspruch 1, **dadurch gekennzeichnet, daß** die Umsetzung in isomeren C₇-C₉-Alkoholgemischen oder C₈-Alkoholgemischen oder C₉-Alkoholgemischen erfolgt.

4. Verfahren nach einem oder mehreren der Ansprüche 1 bis 3, **dadurch gekennzeichnet, daß** die Umsetzung bei einer Temperatur von 140 bis 180 °C erfolgt.

5. Verfahren nach einem oder mehreren der Ansprüche 1 bis 4, **dadurch gekennzeichnet, daß** man das Ethylenoxid dem oder den vorgelegten C₃-C₉-Alkohol(en) und dem Ethylendiamin in einem Zeitraum von 5 bis 60 Minuten zudosiert.

6. Verfahren nach Anspruch 5, **dadurch gekennzeichnet, daß** man das Ethylenoxid in einem Zeitraum von 15 bis 25 Minuten zudosiert.

7. Verfahren nach einem oder mehreren der Ansprüche 1 bis 6, **dadurch gekennzeichnet, daß** man die Menge an gesättigtem C₃-C₉-Alkohol so wählt, daß die Konzentration des Endproduktes nach der Umsetzung 5 bis 60% beträgt.

8. Verfahren nach Anspruch 7, **dadurch gekennzeichnet, daß** die Konzentration des Endproduktes nach der Umsetzung 20 bis 40% beträgt.

## Claims

1. A process for the preparation of N,N,N',N'-tetra-(2-hydroxyethyl)-ethylene diamine by means of the reaction of ethylene diamine with ethylene oxide in a solvent, **characterised in that** ethylene diamine is reacted with ethylene oxide in the absence of water in one or more saturated C₃-C₉ alcohol(s) at a temperature of 120 to 220°C and a pressure of 2 to 60 bar in a molar ratio of 1:4.

2. A process according to claim 1, **characterised in that** the reaction takes place in isopropanol.

3. A process according to claim 1, **characterised in that** the reaction takes place in isomeric C₇-C₉ alcohol mixtures or C₈ alcohol mixtures or C₉ alcohol mixtures.

4. A process according to one or more of claims 1 to 3, **characterised in that** the reaction takes place at a temperature of 140 to 180°C.

5. A process according to one or more of claims 1 to 4, **characterised in that** the ethylene oxide is dosed to the presented C₃-C₉ alcohol(s) and the ethylene diamine in a period of time of 5 to 60 minutes.

6. A process according to claim 5, **characterised in that** the ethylene oxide is dosed in a period of time of 15 to 25 minutes.

7. A process according to one or more of claims 1 to 6, **characterised in that** the amount of saturated C₃-C₉ alcohol is selected such that the concentration of the end product after the reaction is 5 to 60%.

8. A process according to claim 7, **characterised in that** the concentration of the end product after the reaction is 20 to 40%.

## Revendications

1. Procédé de fabrication de N,N,N',N'-tétra-(2-hydroxyéthyl)-éthylène diamine par réaction de l'éthylène diamine avec l'oxyde d'éthylène dans un solvant, **caractérisé en ce que** l'on fait réagir dans un rapport molaire de 1 : 4 l'éthylène diamine avec l'oxyde d'éthylène en l'absence d'eau dans un ou plusieurs alcools saturés en C3 à C9 à une température de 120 à 220 °C et sous une pression de 2 à 60 bar.

2. Procédé selon la revendication 1, **caractérisé en ce que** la réaction s'effectue dans l'isopropanol.

3. Procédé selon la revendication 1, **caractérisé en ce que** la réaction s'effectue dans des mélanges d'isomères d'alcools en C7 à C9, ou d'alcools en C8, ou d'alcools en C9.

4. Procédé selon une des revendications 1 à 3, **caractérisé en ce que** la réaction s'effectue à une température comprise entre 140 et 180 °C.

5. Procédé selon une des revendications 1 à 4, **caractérisé en ce que** l'on dose l'oxyde d'éthylène dans le ou les alcools en C3 à C9 et dans l'éthylène diamine en l'espace de 5 à 60 minutes.

6. Procédé selon la revendication 5, **caractérisé en ce que** l'on dose l'oxyde d'éthylène en l'espace de 15 à 25 minutes.

7. Procédé selon une des revendications 1 à 6, **caractérisé en ce que** l'on choisit la quantité d'alcool saturé en C3 à C9 de telle sorte que la concentration du produit final après la réaction soit de 5 à 60 %.

8. Procédé selon la revendication 7, **caractérisé en ce que** la concentration du produit final après la réaction est de 20 à 40 %.
